# FASCICULE DE BREVET EUROPEEN

(11) **EP 1 131 124 B1**
(45) Date de publication et mention de la délivrance du brevet: **18.01.2006**
(21) Numéro de dépôt: 99943011.9
(22) Date de dépôt: 21.09.1999
(51) Int. Cl.: A61M 25/00, A61M 5/30, A61M 5/44

(54) **DISPOSITIF DESTINE A ASSURER LA DELIVRANCE D'UNE SUBSTANCE ACTIVE DIRECTEMENT AU SEIN D'UN TISSU CELLULAIRE, MOYEN D'IMPLANTATION DU DISPOSITIF ET APPAREILS DESTINES A L'INJECTION DE SUBSTANCE ACTIVE DANS LEDIT DISPOSITIF**
VORRICHTUNG ZUM EINFÜHREN EINES MEDIKAMENTS IN EINER GEWEBSMEMBRAN, IMPLANTATIONSVORRICHTUNG UND INJEKTIONSVORRICHTUNG
DEVICE FOR DIRECTLY DELIVERING AN ACTIVE SUBSTANCE WITHIN A CELL TISSUE, MEANS FOR IMPLANTING SAID DEVICE AND APPLIANCES FOR INJECTING ACTIVE SUBSTANCE INTO SAID DEVICE

(30) Priorité: 17.11.1998 FR 9814582; 19.03.1999 FR 9903624
(43) Date de publication de la demande: 12.09.2001
(73) Titulaire: Mehier, Henri, F-69002 Lyon (FR)
(72) Inventeur: Mehier, Henri, F-69002 Lyon (FR)
(74) Mandataire: Vuillermoz, Bruno
(86) Numéro de dépôt international: PCT/FR1999/002234
(87) Numéro de publication internationale: WO 2000/029055

(56) Documents cités:
- WO-A-89/02290
- WO-A-96/20022
- WO-A-96/36381
- DE-A- 2 813 750
- US-A- 4 377 165
- US-A- 5 254 094
- US-A- 5 364 374
- US-A- 5 381 510
- US-A- 5 569 197

## Description

L'invention concerne un dispositif destiné à assurer la délivrance d'une substance active directement au sein de tout ou partie d'un tissu cellulaire humain ou animal. Elle se rapporte également au moyen d'implantation de ce dispositif au sein du tissu, de même qu'aux appareils destinés à l'injection de la substance active dans ledit dispositif.

Dans la suite de la description et dans les revendications, par l'expression "substance active", on désigne toute substance chimiquement ou physiquement active et plus largement, toute substance susceptible d'être introduite dans l'organisme qu'elle soit à visée diagnostic, thérapeutique, ou même cosmétique.

De même, par l'expression « tissu cellulaire humain ou animal », on désigne tout organe ou partie d'organe.

Dans le cadre des traitements anti-cancéreux par rayonnement ionisant, plusieurs solutions ont été proposées visant à concentrer le principe actif au niveau des cellules à traiter du patient en limitant la destruction des cellules saines.

On a ainsi proposé dans le document WO 97/18011 une installation pour concentrer un principe actif associé à des vecteurs magnétiques au niveau des cellules à traiter d'un patient. L'installation mise en oeuvre est apte à créer un gradient de champ magnétique focalisé, puis par augmentation de _l'intensité du champs magnétique, à faire passer les vecteurs magnétiques de l'état non-magnétisé à l'état magnétisé, afin de créer une agrégation durable de ceux-ci dans la zone des cellules à traiter.

Toutefois, même si cette installation permet de limiter, voire de supprimer l'irradiation des cellules saines, elle reste néanmoins lourde à mettre en oeuvre.

On a également proposé dans le document JPS Volume 71, Numéro 4, April 82 (page 382) une méthode consistant à injecter par voie artérielle un principe actif associé à des vecteurs magnétiques soumis à un champ magnétique externe, lequel est ensuite focalisé au niveau des cellules à traiter. Toutefois, on observe qu'une partie importante des vecteurs magnétiques s'agrège et reste bloquée dans les vaisseaux du réseau sanguin irradiant ainsi un grand nombre de cellules saines.

Toujours dans le cadre de la cancérothérapie, on met avantageusement en oeuvre, en complément des principes actifs classiques, un traitement des cellules cancéreuses par la chaleur, dans la mesure où celle-ci provoque un effet cytolytique immédiat sur lesdites cellules. En pratique, l'échauffement des cellules cancéreuses est obtenue par un traitement par micro-ondes. Cette thérapie permet ainsi d'élever la température de façon localisée et ainsi provoquer une hyperthermie.

Le traitement par la chaleur est également mis en oeuvre pour stopper les hémorragies, notamment celles susceptibles d'apparaître dans le cadre d'opérations chirurgicales, et ainsi provoquer une hémostase. En pratique, l'hémostase est obtenue par électro-coagulation au moyen d'un bistouri électrique.

Le problème de la concentration du principe actif au niveau des cellules à traiter ne se limite pas au traitement anti-cancéreux, mais concerne également bon nombre de thérapies que ce soit chimiothérapie, antibiothérapie, etc.

Dans le document US-A-5 569 197, on a proposé de concentrer un principe actif par voie endoluminale, au niveau de plaques d'athéromes formées sur la paroi internes des artères. Le dispositif mis en oeuvre correspond à un tube réalisé en un matériau dit "superélastique" de diamètre externe supérieure à 250 micromètres, avantageusement 360 micromètres, dont les parois au niveau de l'extrémité distale dudit tube sont munies de perforations. En outre, l'extrémité distale du tube est ouverte et peut être équipée d'un fil présentant lui-même une ouverture destinée à assurer la délivrance du principe actif. Le fil souple a pour fonction de guider le tube dans les petits vaisseaux. Le principe actif, se présentant sous forme liquide, est délivré exclusivement au niveau des plaques d'athérome par infusion à une pression n'excédant pas 4 atmosphères, c'est à dire légèrement supérieure à 4 bars.

Tout d'abord, le dispositif décrit dans ce document ne permet d'obtenir un effet thérapeutique qu'au niveau des organes vascularisés dans la mesure où le dispositif proposé est utilisé exclusivement par voie endoluminale. De plus, la concentration en principe actif au niveau de la zone à traiter n'est augmentée que de façon temporaire, le reste du principe actif diffusant dans l'organisme.

Document US-A-5 702 372 décrit un dispositif comme récité dans le préambule de la revendication 1.

En d'autres termes, le premier problème que se propose de résoudre l'invention est de fournir un dispositif apte à assurer la délivrance d'une substance active au niveau de n'importe quel organe.

Un second problème que se propose de résoudre l'invention est de fournir un dispositif apte à permettre une libération homogène de la substance active in situ, exclusivement au niveau de tout ou partie du tissu cellulaire à traiter, sans aucune diffusion dans l'organisme.

Un autre problème que se propose de résoudre l'invention est de fournir un dispositif susceptible de dispenser tout type de principe actif que ce soit principe actif chimique ou principe actif physique et sous n'importe quelle forme liquide, solide, et même vapeur.

Pour ce faire, la présente invention propose un dispositif apte à assurer la délivrance d'une substance active directement au sein de tout ou partie d'un tissu cellulaire humain ou animal.

Ce dispositif se caractérise en ce qu'il se présente sous forme d'un tube creux dont les parois en contact avec ledit tissu sont munies d'au moins une perforation et dont l'extrémité distale est obturée, tandis que l'extrémité proximale est conformée de sorte à recevoir des moyens de fermeture amovible, ledit tube étant apte à supporter une pression d'au moins 50 bars.

Autrement dit, l'idée du Demandeur est non pas d'assurer la délivrance d'un principe actif par voie endoluminale, mais directement au niveau du tissu cellulaire par injection dudit principe actif au niveau d'une zone cible à pression élevée d'au moins 50 bars, permettant d'obtenir un maximum d'efficacité, y compris dans l'épaisseur du tissu.

Face à ce problème, le Demandeur a développé un dispositif apte à supporter une pression élevée permettant d'injecter le principe actif directement dans les tissus à une profondeur variant en fonction de la pression mise en oeuvre. En outre et compte tenu de la pression élevée, le dispositif peut être traversé par des principes actifs sous forme liquide ou solide, ou même vapeur.

En pratique, les parois perforées du tube creux sont positionnées au niveau de l'organe cible à traiter, tandis que le reste du tube relie l'extérieur de l'organisme, le tube restant ainsi en position tout au long du traitement. Dès lors, le tube reçoit, via son extrémité proximale, la substance active qu'il délivre via ses perforations exclusivement au niveau des tissus à traiter.

Comme il sera expliqué par la suite, la substance active doit être animée d'une énergie suffisante, pour pouvoir être propulsée à travers les perforations, puis pénétrer ensuite efficacement dans les tissus à traiter.

Parallèlement, le tube doit garder une certaine souplesse pour faciliter sa mise en place au sein de l'organisme et plus particulièrement, au niveau des tissus.

Pour répondre à ces exigences, le choix du diamètre et de l'épaisseur du tube résultent d'un compromis entre souplesse, résistance à la pression et étirement dudit tube.

Lorsqu'il s'agit d'injecter une substance chimiquement active dans les tissus, la pression peut atteindre 3000 bars, voire même 10000 bars, si l'on souhaite injecter une poudre.

Dans un premier mode de réalisation, pour résister à une telle pression tout en gardant une certaine souplesse, le diamètre extérieur du tube est compris entre 100 et 250 micromètres; tandis que le diamètre intérieur est compris entre 50 et 150 micromètres.

Pour un diamètre extérieur supérieur à 250 micromètres, il est nécessaire d'augmenter l'épaisseur du tube pour résister à la pression, de sorte que la flexibilité disparaît. Au contraire, pour un diamètre intérieur inférieur à 50 micromètres, la perte d'énergie de la substance active au cours de son déplacement dans le tube est trop importante, de sorte que la substance active ne peut être propulsée dans le tissu à traiter.

Avantageusement, le diamètre extérieur du tube est égal à 200 micromètres, tandis que le diamètre intérieur du tube est égal à 100 micromètres.

De même, les perforations ménagées sur les parois du tube sont sensiblement circulaires et ont un diamètre compris entre 30 et 70 micromètres, avantageusement 50 micromètres. En pratique, ces perforations sont effectuées au laser.

Pour un diamètre inférieur à 30 micromètres, les perforations sont trop petites pour permettre le passage de la substance active. Au contraire, pour un diamètre supérieur à 70 micromètres, l'ouverture est trop importante pour assurer une résistance satisfaisante du tube.

En outre, pour résister à une pression interne pouvant atteindre 10000 bars, le matériau utilisé pour la fabrication du tube est choisi dans le groupe comprenant l'acier inoxydable et le titane. De la sorte, le rayon de courbure du tube obtenu est de l'ordre de 1 centimètre et ce, sans déformation permanente.

Cependant, on pourra utiliser n'importe quel matériau susceptible de supporter des pressions élevées de l'ordre de 3 à 10 000 bars. -

Par ailleurs, lorsqu'il s'agit d'injecter une substance physiquement active, telle que de la vapeur d'eau ou encore de la vapeur d'eau oxygénée, la pression est d'au moins 50 bars, en pratique égale à 200 bars.

De plus, en utilisant en tant qu'actif l'eau oxygénée, on obtient un effet thérapeutique supplémentaire grâce à la transformation d'eau oxygénée, notamment en oxygène naissant.

Avantageusement, on utilise une solution aqueuse d'eau oxygénée dont la concentration est comprise en volume entre 40 et 60 %.

Dans ce cas, compte-tenu de la pression plus faible, le diamètre extérieur du tube peut être plus élevé sans toutefois que ce dernier perde sa souplesse.

Dès lors et selon un second mode de réalisation, le diamètre extérieur du tube creux est compris entre 300 et 700 micromètres, tandis que le diamètre intérieur est compris entre 100 et 300 micromètres.

Pour un diamètre extérieur supérieur à 700 micromètres, il est nécessaire d'augmenter l'épaisseur du tube pour résister à la pression, de sorte que la flexibilité disparaît. Au contraire, pour un diamètre extérieur inférieur à 100 micromètres, la perte d'énergie de la vapeur au cours de son déplacement dans le tube est trop importante, de sorte qu'elle ne peut être propulsée dans l'organe à traiter.

Par ailleurs, selon ce mode de réalisation, les perforations ménagées sur les parois sont sensiblement circulaires et ont un diamètre compris entre 100 et 200 micromètres, avantageusement 150 micromètres.

Pour un diamètre inférieur à 100 micromètres, les perforations sont trop petites pour permettre le passage d'une quantité efficace de la vapeur. Au contraire, pour un diamètre supérieur à 200 micromètres, l'ouverture est trop importante pour assurer une résistance satisfaisante du tube.

Pour résister à une pression en pratique de l'ordre de 200 bars, tout en gardant un maximum de souplesse et de résistance à la chaleur, le tube est avantageusement réalisé en polytétrafluoroéthylène (PTFE) ou Teflon®. Bien entendu, tout matériau équivalent en terme de résistance à la pression, peut être envisagé.

Bien que l'injection de vapeur d'eau ou d'eau oxygénée puisse être effectuée dans le tube présentant les caractéristiques décrites ci-avant, elle peut également l'être dans un tube identique à celui mis en oeuvre pour l'injection de principe actif chimique, à une pression moindre de l'ordre de 200 bars. Dans ce cas, le médecin disposera d'un seul dispositif qu'il pourra utiliser in situ pour l'administration soit de principe actif chimique, soit de principe actif physique sous forme de vapeur.

Selon ce mode de réalisation, la partie du tube, qui n'est pas en contact avec le tissu à traiter, est gainée d'un matériau isolant, souple et résistant à la chaleur.

Pour obtenir une distribution homogène du principe actif, les perforations sont ménagées en spirale tout autour de la partie du tube destiné à être au contact de l'organe à traiter, avec un pas compris entre 0,1 et 2 centimètres.

Par ailleurs, comme déjà dit, l'extrémité proximale du tube présente des moyens de fermeture amovible. En pratique, mais de façon non limitative, ces moyens peuvent se présenter sous forme d'un bouchon fileté destiné à coopérer avec un taraudage correspondant ménagé à l'extrémité proximale du tube.

De plus, pour participer à la connexion entre le tube et l'appareil destiné à l'injection de substance active décrit par la suite, ledit tube présente un joint conique fixé au voisinage de son extrémité proximale.

Comme déjà dit, la mise en oeuvre du dispositif de l'invention ne se limite pas à la cancérologie mais concerne également l'antibiothérapie. Dans ce cadre, le tube de l'invention trouve une application particulièrement intéressante en orthopédie. En effet, l'implantation chirurgicale d'une prothèse peut donner naissance à une infection qu'il est bien souvent difficile de traiter localement. Dans un tel cas de figure, le chirurgien pourra implanter un élément d'ostéosynthèse tel que prothèse, clou etc... équipé par tout moyen connu du tube creux de l'invention, lequel restera en relation avec le milieu extérieur à l'organisme.

A titre d'exemple, le microtube peut être logé dans un sillon en spirale creusé à la surface du matériel implanté dans l'os. Dans ce cas, le principe actif injecté peut être un antibiotioque en cas d'infection osseuse, ou un produit de scellement en cas de descellement d'une prothèse.

L'invention concerne donc également un élément d'ostéosynthèse équipée du tube de l'invention.

L'invention vise aussi un moyen d'implantation du dispositif précédemment décrit au sein d'un organe cible humain ou animal. Cette implantation peut être effectuée par divers moyens.

Selon un premier procédé, l'implantation du tube se fait par ponction guidée par imagerie au moyen d'une aiguille. Dans ce cas, le moyen d'implantation se présente donc sous forme d'une aiguille destinée à recevoir le tube et présentant sur toute ou partie de sa longueur une fente destinée à assurer le dégagement et le maintien en position du tube après retrait de l'aiguille.

Selon un autre procédé, l'implantation est effectuée chirurgicalement. Dans ce cas, le moyen d'implantation se présente sous forme d'une aiguille fixée à l'extrémité distale du tube creux, la fixation étant réalisée par tout moyen connu.

Selon cette technique, si l'on souhaite implanter ledit tube tout autour d'un organe cible, par exemple le foie, on prévoit alors des perforations sur des longueurs du tube égales à la longueur de l'aiguille séparées par des longueurs non perforées, qui peuvent être repérées notamment par des dépôts d'or pour la reprise de l'aiguille, les longueurs non perforées se trouvant alors en dehors de l'organe à traiter.

Selon un autre procédé, l'implantation est effectuée par voie endovasculaire, le tube constituant alors le cathéter.

L'invention concerne aussi l'appareil destiné à l'injection de substance active dans le dispositif.

Cependant, la configuration de l'appareil pourra varier en fonction de la pression à laquelle la substance active est injectée.

Ainsi, lorsque la substance active est injectée à très haute pression, de l'ordre de 3 à 10 000 bars, l'appareil se caractérise en ce qu'il présente :
- d'une part, un moyen de stockage de substance active apte à être connecté à l'extrémité proximale du tube ;
- d'autre part, des moyens aptes à permettre la propulsion de la substance active à travers le tube.

En pratique, l'extrémité proximale du tube extérieure à l'organisme est connectée directement au moyen de stockage, lequel est soumis à des moyens qui permettront d'injecter la substance active dans le tube avec une pression telle qu'elle sera propulsée à travers les perforations de sorte à parvenir jusqu'à la zone cible à traiter.

Selon une première forme de réalisation, le moyen de stockage de substance active se présente sous forme d'un cylindre dont l'axe central est évidé pour former un réservoir cylindrique destiné à recevoir la substance active et dont l'une des extrémités est destinée à être connectée directement à l'extrémité proximale du tube, tandis que l'autre extrémité est obturée par un bouchon étanche mobile axialement sous l'action de la tige d'un piston.

Parallèlement et selon une autre caractéristique de l'appareil, les moyens aptes à permettre l'injection de principe actif dans le tube se présentent sous forme d'une masse destinée à être propulsée contre le piston, lequel agira par l'intermédiaire de sa tige sur le bouchon mobile étanche entraînant ainsi l'éjection de substance active dans le tube.

Dans une autre forme de réalisation, la substance active est stockée dans une ampoule à paroi déformable, laquelle est positionnée dans le réservoir cylindrique décrit ci-avant, l'extrémité de l'ampoule étant connectée par tout moyen connu à l'extrémité proximale du tube. Ce mode de réalisation permet ainsi de disposer de doses de substance active prêtes à l'emploi.

Par ailleurs, la propulsion de la masse peut être effectuée par divers moyens, notamment au moyen d'un système pneumatique ou encore d'un système électromagnétique.

Par ailleurs, lorsque la substance active est une substance physiquement active, notamment de la vapeur d'eau ou d'eau oxygénée, l'appareil se caractérise en ce qu'il présente :
- un moyen de chauffage de la substance active,
- un moyen d'amenée de la substance active au moyen de chauffage,
- un moyen de transmission de la substance active chauffée dans le tube.

Comme déjà dit, la substance physiquement active mise en oeuvre se présente en pratique sous forme de vapeur d'eau ou de vapeur d'eau oxygénée.

En pratique, le moyen d'amenée de la substance active et le moyen de transmission de la substance active chauffée jusqu'au tube de l'invention se présentent sous forme d'un tube inox enroulé autour d'une bobine d'aluminium.

De plus, le moyen de chauffage de la substance active se présente sous forme d'une résistance électrique enserrant l'ensemble bobine-tube.

Selon une autre caractéristique, la bobine est équipée d'une sonde platine reliée au système électrique permettant ainsi de réguler la température.

En pratique, l'eau ou l'eau oxygénée est injectée dans le tube inox à une pression minimum de 200 bars et ressort par le même tube inox après avoir été chauffée par la bobine à une température voisine de 400° C.

L'invention concerne enfin un procédé d'administration d'une substance active directement au sein de tout ou partie d'un tissu humain ou animal selon lequel :
- on localise tout d'abord le tissu à traiter,
- on introduit ensuite dans l'organisme, jusqu'au niveau du tissu à traiter, un tube creux, dont les parois en contact avec ledit tissu sont munies d'au moins une perforation et dont l'extrémité distale est obturée, l'extrémité proximale dudit tube reliant l'extérieur de l'organisme,
- puis, on injecte sous pression d'au moins 50 bars, la substance active dans ledit tube, de sorte qu'elle soit délivrée dans les tissus via les perforations,
- on bouche enfin l'extrémité proximale du tube,
- en fin de traitement, on retire le tube.

Comme déjà dit, l'introduction du tube creux dans l'organisme se fait par tout moyen connu et en particulier par les moyens d'implantation décrits ci-avant.

De même, l'injection de substance active est réalisée par un appareil du type de celui précédemment décrit ou tout moyen équivalent, l'appareil étant désolidarisé du tube après chaque injection.

Bien entendu, le même tube peut être utilisé au choix pour l'injection de substance active chimique ou physique. En pratique, le chirurgien pourra, lors de l' implantation du tube, provoquer une hémostase si besoin est, par injection de vapeur.

Par ailleurs, la substance active peut revêtir différentes formes, notamment liquide, ou encore solide, par exemple sous forme de nanocapsules, nanoparticules ou microparticules. On peut ainsi envisager tous types de substances actives, que ce soient celles utilisées en chimiothérapie ou encore en antibiothérapie, de même que les anti-inflammatoires et les produits radioactifs à visée thérapeutique, et ce de façon non limitative.

Dans une forme de réalisation avantageuse, la substance active peut être associée à des nanoparticules magnétiques de ferrite de taille comprise entre 100 et 1000 nanomètres.

Il s'ensuit que lors de l'injection de la substance active à travers le tube, l'énergie communiquée aux nanoparticules magnétiques fait qu'elles se comportent de façon indépendante les unes des autres, leur attraction mutuelle magnétique devenant en effet négligeable par rapport à leur énergie cinétique. En revanche, après injection, c'est-à-dire in situ, l'attraction magnétique favorise le regroupement des nanoparticules sous forme d'amas de tailles d'environ 50 micromètres, dans l'organe ou la zone d'organe à traiter.

Dans le cas d'un principe actif radioactif, ledit principe actif radioactif peut revêtir deux formes différentes :
- soit, il est constitué d'isotopes radioactifs et greffé sur les particules magnétiques ;
- soit, il est inclus dans la particule magnétique et est constitué d'isotopes radioactifs des éléments magnétiques formant les particules magnétiques.

Avantageusement, le produit radioactif peut être émetteur de rayonnement α, β et γ à visée thérapeutique, de préférence de faible énergie, pour obtenir une irradiation la plus locale possible. Il peut être également utile d'associer un émetteur γ d'énergie comprise entre 100 et 150 kiloélectronvolts (Kev) ou émetteur β+ pour visualiser la localisation des nanoparticules à l'aide d'une γ-caméra. Ceci permet en outre de faciliter le calcul de la dose d'irradiation.

Comme déjà dit, on peut utiliser en tant que particules magnétiques des nanoparticules de ferrite.

Dans ce cas, le produit stable donnant le produit radioactif par irradiation par neutrons ou particules chargées est incorporé lors de la fabrication de nanoparticules de ferrite, les composants de la ferrite donnant après irradiation, des produits radioactifs parasites de très courte période, disparaissant donc très vite. De la sorte, seule persiste la radioactivité de l'élément radioactif thérapeutique choisi.

Dans une autre forme de réalisation, on peut associer une substance active à du mercure (Hg) liquide ou en amalgame sous forme de nanoparticules. En effet, lors de l'injection, le mercure liquide prend la forme de micro-gouttes dont l'énergie cinétique est élevée en raison de sa forte densité. In situ, c'est-à-dire au niveau de l'organe, la tension superficielle importante du mercure favorise le regroupement des micro-gouttes en billes plus grosses fixant ainsi la substance active dans l'organe à traiter.

De plus, le mercure possède un isotope radioactif (Hg 197) bien adapté à la thérapie. De la sorte, le principe actif Hg 197 est inclu dans la nanoparticule de mercure. En outre et comme déjà dit, le mercure réalise des amalgames avec la plupart des métaux, ce qui permet donc de fixer d'autres produits radioactifs métalliques sous forme de traces, le mercure restant liquide.

Les avantages de l'invention ressortiront bien de l'exemple de réalisation ci-après à l'appui des figures annexées.

La figure 1 est une représentation du dispositif de l'invention.

La figure 2 est une représentation d'un premier moyen d'implantation.

La figure 3 est une représentation d'un second moyen d'implantation.

La figure 4 est une représentation d'un appareil destiné à l'injection du principe actif dans le tube lorsque ledit principe actif est injecté sous très haute pression de l'ordre de 3 000 à 10 000 bars.

La figure 5 est une représentation d'un appareil du même type que celui de la figure 4 selon une autre forme dé réalisation.

La figure 6 est une représentation de la figure 5 selon l'axe II.

La figure 7 est une représentation en coupe d'un appareil destiné à l'injection de principe actif lorsque ledit principe actif est injecté à une pression de l'ordre de 200 bars.

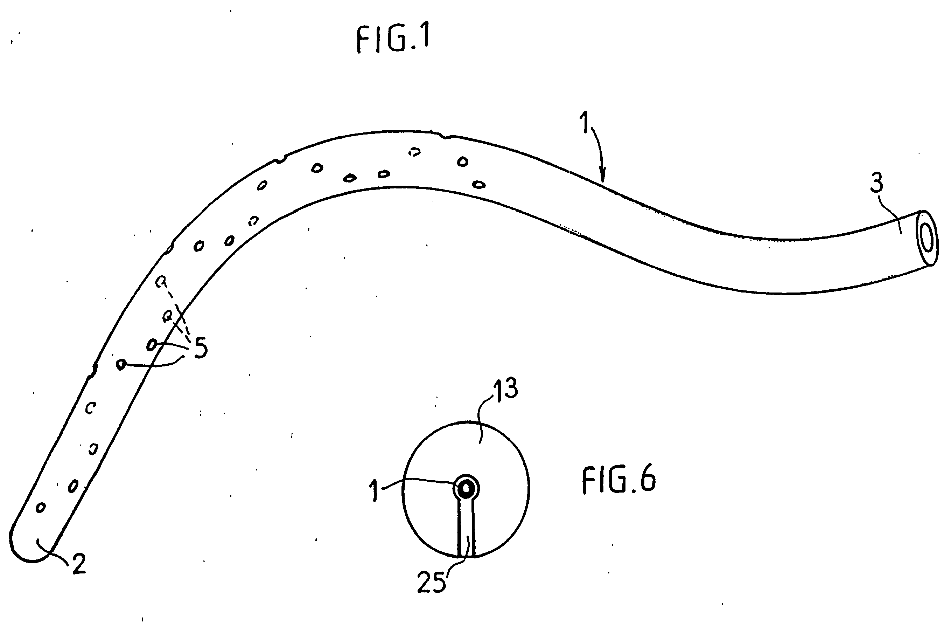

Sur la figure 1, on a représenté le tube creux objet de l'invention désigné par la référence générale 1. Le tube creux présente une extrémité distale (2) obturée, tandis que l'extrémité proximale (3) est conformée de sorte à recevoir un moyen de fermeture amovible, par exemple un bouchon. En pratique, l'extrémité proximale du tube présente un taraudage (non représenté) destiné à coopérer par vissage avec le filetage du bouchon.

Le diamètre varie en fonction de la pression à laquelle il est soumis. Ainsi, si la pression est de l'ordre de 3 000 bars et plus, le diamètre extérieur du tube est de 200 micromètres, tandis que son diamètre intérieur est choisi égal à 100 micromètres. Si la pression est de l'ordre de 200 bars, le diamètre extérieur est de 400 micromètres, tandis que son diamètre interne est choisi égal à 150 micromètres Bien entendu, la longueur du tube varie en fonction de la distance entre le point d'introduction du tube dans l'organisme et l'organe à traiter.

Par ailleurs, pour permettre une mise à disposition du principe actif homogène, le tube présente des perforations (5) ménagées au laser en spirale sur la partie du tube destinée à être au contact direct du tissu à traiter et ce, avec un pas par exemple égal à 0,5 centimètres.

De plus, pour favoriser la connexion entre le tube et les appareils d'injection décrits par la suite, le tube présente au voisinage de son extrémité proximale un joint tronconique. Il est percé en son centre d'un orifice correspondant au diamètre du tube.

Pour que le tube garde toute sa souplesse et puisse résister à la forte pression, il est fabriqué en un acier inoxydable de qualité A 304 (norme internationale).

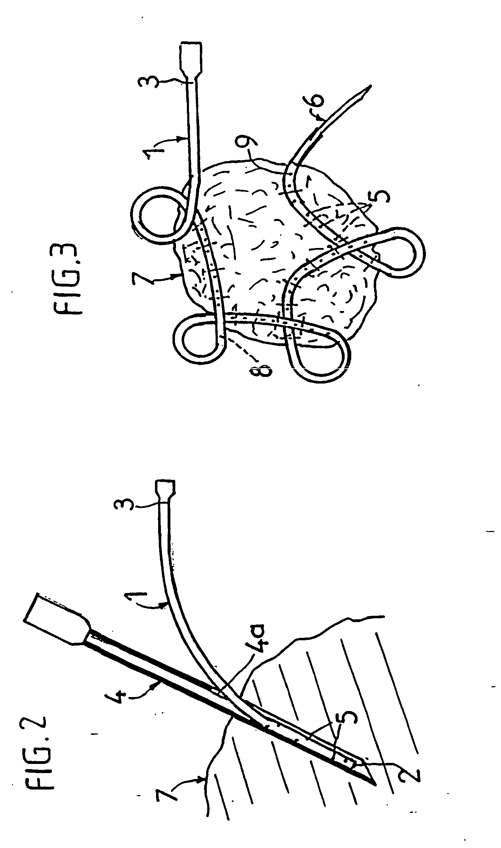

Sur la figure 2, on a représenté un premier moyen d'implantation du tube au sein d'un organe.

Dans cette forme de réalisation, l'implantation est réalisée par ponction guidée au moyen d'une aiguille (4). L'aiguille est destinée à recevoir le tube et présente sur tout ou partie de sa longueur une fente (4a) destinée à assurer le dégagement et le maintien en position du tube après retrait de l'aiguille.

Selon un autre mode de réalisation, le moyen d'implantation se présente sous forme d'une aiguille (6) agencée à l'extrémité distale (2) du tube (1) par tout moyen connu, notamment par sertissage. Le tube est alors "cousu" tout autour de l'organe à traiter (7). Dans ce cas, et pour obtenir une libération de la substance active exclusivement au niveau de l'organe, les perforations sont ménagées uniquement sur les parties (9) au contact de l'organe, les parties (8) du tube restant à l'extérieur de l'organe et étant obturées interdisant ainsi la libération de la substance active à ce niveau.

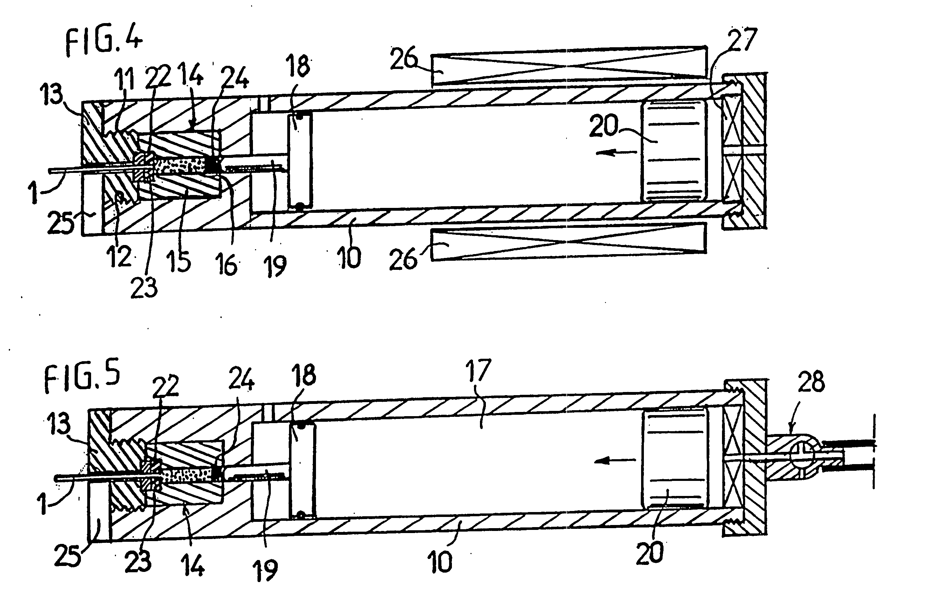

On a représenté sur la figure 4 un premier mode de réalisation l'appareil destiné à l'injection de substance active dans le tube (1) sous haute pression de l'ordre de 3 000 bars et plus. Cet appareil se présente sous la forme générale d'un cylindre (10). Ce cylindre est muni à l'une des ses extrémités d'un taraudage (11) destiné à coopérer avec le filetage (12) d'une visse (13). L'appareil de forme cylindrique (10) présente en outre deux compartiments :
- un premier compartiment (14) situé à proximité du bouchon (13) et destiné à recevoir un cylindre (15) constituant moyen de stockage de la substance active, dont l'axe central est évidé, de sorte à constituer un réservoir cylindrique (16) destiné à recevoir la substance active proprement dite ;
- un second compartiment cylindrique (17) creux formant chambre dans laquelle est mû en translation un piston (18) muni d'une tige (19) sous l'action d'une masse (20).

Le cylindre (15) présente en outre à son extrémité voisine du bouchon (13) une échancrure (22) destinée à recevoir une rondelle, notamment élastomère (23). A son extrémité opposée, le cylindre (15) présente un bouchon (24) destiné à obturer le réservoir cylindrique (16).

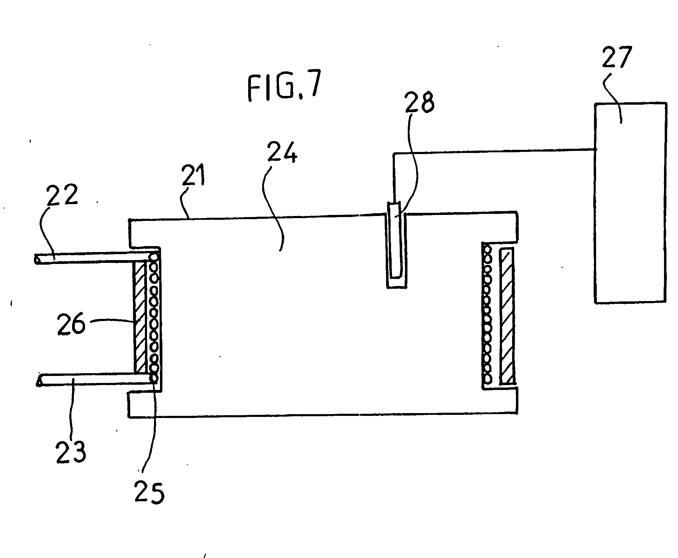

Comme représenté sur la même figure, le bouchon (13) est percé de part en part selon son axe central, de sorte à permettre le passage du tube (1). Pour simplifier la mise en place du tube (1) à travers le bouchon (13), celui-ci présente longitudinalement une fente radiale (25), (voir figure 6).

Comme déjà dit, la chambre (17) renferme une masse (20) destinée à être propulsée contre le piston (18). La masse (20) est maintenue immobile au moyen d'un aimant (27) positionné dans le fond du compartiment (17). La masse (20) peut être propulsée par divers moyens.

Un premier moyen consiste à créer un champ magnétique au moyen d'un bobinage (26).

La propulsion de la masse peut être également effectuée au moyen d'un système d'air comprimé (28), comme représenté sur la figure 5.

Sur la figure 7, on a représenté un appareil destiné à l'injection de substance active sous une pression de l'ordre de 200 bars. Comme déjà dit, dans ce cas, la substance injectée est une substance physiquement active, notamment sous forme de vapeur d'eau ou d'eau oxygénée.

Selon ce mode de réalisation, l'appareil présente :
- un moyen de chauffage (21) de l'eau ou de l'eau oxygénée ;
- un moyen d'amenée de l'eau ou de l'eau oxygénée (22) ;
- et un moyen de transmission de l'eau ou de l'eau oxygénée vaporisée (23).

Le moyen d'amenée de l'eau ou de l'eau oxygénée et les moyens de transmission de l'eau ou de l'eau oxygénée vaporisés se présentent sous la forme d'une bobine d'aluminium (24) de diamètre égal à 100 millimètres et de hauteur égale à 60 millimètres, enroulée d'un tube inox (25) de diamètre extérieur égal à 1,6 millimètres et de diamètre interne égal à 0,3 millimètre, de longueur comprise entre 1500 et 3000 millimètres. L'extrémité (22) du tube (25) correspond au moyen d'amenée de la substance active et l'extrémité (23) au moyen de transmission de la vapeur au tube de l'invention.

L'ensemble bobine (24) et tube inox (25) est entouré du moyen de chauffage proprement dit sous forme d'une résistance (26) électrique.

Par ailleurs, la bobine (24) est équipée d'une sonde (28) reliée à l'alimentation électrique (27) laquelle régule la température de la bobine.

En pratique et comme déjà dit, l'eau est injectée à une pression de 200 bars dans le tube inox, l'eau étant ensuite chauffée au moyen de la bobine à une température de 400° C, correspondant à une tension de saturation de vapeur égale à 200 bars, la vapeur étant réinjectée dans le tube objet de l'invention à une pression de 200 bars.

En pratique, le chirurgien va d'abord délimiter le tissu ou la partie de tissu à traiter, et donc son positionnement. Il détermine ainsi la longueur du tube nécessaire pour atteindre ledit tissu depuis le point d'introduction du tube dans l'organisme. Parallèlement, il choisit le tube adéquat, c'est-à-dire celui présentant des perforations positionnées de telle sorte que la substance active soit libérée exclusivement dans le tissu ou partie de tissu à traiter. Il positionne ensuite le tube au niveau de l'organe à traiter, soit par ponction guidée, soit chirurgicalement au moyen d'une aiguille, ou encore par voie endovasculaire. La longueur du tube est choisie de sorte que seule une faible longueur, de l'ordre d'une dizaine de centimètres, reste en dehors de l'organisme. L'extrémité proximale dudit tube est alors connectée aux appareils ci-avant décrits.

S'agissant de la précision de ces deux appareils, le tube est introduit dans la fente radiale ménagée sur le bouchon de l'appareil. Le tube est alors connecté à la cavité renfermant la substance active par vissage du bouchon, lequel entraîne un contact étroit entre la rondelle acier et la rondelle en élastomère.

Une fois le tube connecté au moyen de stockage, on propulse la masse sur le piston, de sorte à ce que la substance active soit injectée directement dans le tube et délivrée exclusivement au niveau de l'organe à traiter.

S'agissant du second appareil, le tube de l'invention est directement connecté à l'extrémité du tube inox par tout moyen connu.

Bien entendu et comme déjà dit, le tube peut rester positionné au niveau de l'organe durant toute la durée du traitement. Dans ce cas, après chaque injection de substance active, on referme le tube. En fin de traitement, le tube sera retiré.

Les avantages de l'invention ressortent bien de la description. On notera en particulier la simplicité du matériel mis en oeuvre et l'efficacité du traitement dans la mesure où la substance active peut être administrée in situ exclusivement dans la zone à traiter et en profondeur.

## Revendications

1. Dispositif apte à assurer la délivrance d'une substance active directement au sein de tout ou partie d'un tissu cellulaire humain ou animal, se présentant sous forme d'un tube creux (1), muni de perforations (5) ménagées sur toute la longueur des parois destinées à être au contact du tissu et dont l'extrémité distale (2) est obturée, tandis que l'extrémité proximale (3) est conformée de sorte à recevoir des moyens de fermeture amovible, **caractérisé en ce que** ledit tube est apte à supporter une pression d'au moins 50 bars.

2. Dispositif selon la revendication 1 **caractérisé en ce que** le diamètre extérieur du tube (1) est compris entre 100 et 250 micromètres, tandis que le diamètre intérieur du tube (1) est compris entre 50 et 150 micromètres.

3. Dispositif selon l'une des revendications précédentes, **caractérisé en ce que** le diamètre extérieur du tube est égal à 200 micromètres, tandis que le diamètre intérieur du tube est égal à 100 micromètres.

4. Dispositif selon l'une des revendications 2 ou 3, **caractérisé ce que** les perforations (5) sont sensiblement circulaires et ont un diamètre compris entre 30 et 70 micromètres, avantageusement 50 micromètres.

5. Dispositif selon l'une des revendications 2 à 4, **caractérisé en ce que** le matériau constituant le tube est choisi dans le groupe comprenant l'acier inoxydable et le titane.

6. Dispositif selon la revendication 1, **caractérisé en ce que** le diamètre extérieur du tube creux est compris entre 300 et 700 micromètres, tandis que le diamètre intérieur est compris entre 100 et 300 micromètres.

7. Dispositif selon la revendication 6, **caractérisé en ce que** les perforations ménagées sur les parois sont sensiblement circulaires et ont un diamètre compris entre 100 et 200 micromètres, avantageusement 150 micromètres.

8. Dispositif selon l'une des revendications 6 ou 7, **caractérisé en ce que** le tube est réalisé en polytétrafluoroéthylène (PTFE).

9. Dispositif selon l'une des revendications précédentes, **caractérisé en ce que** les perforations (5) sont ménagées en spirale tout autour de la partie du tube destinée à être au contact de l'organe à traiter (7), avec un pas compris entre 0,5 et 2 centimètres.

10. Dispositif selon l'une des revendications précédentes, **caractérisé en ce que** les moyens de fermeture amovibles se présentent sous forme d'un bouchon destiné à être vissé à l'extrémité proximale du tube.

11. Elément d'ostéosynthèse équipée du dispositif objet de l'une des revendications 1 à 10.

12. Appareil pour l'injection de substance active associé au dispositif objet de l'une des revendications 1 à 10, ledit appareil présentant :
• d'une part, un moyen de stockage du principe actif apte à être connecté à l'extrémité proximale du tube ;
• d'autre part, des moyens aptes à permettre l'injection de la substance active à travers le tube (1).

13. Appareil selon la revendication 12, **caractérisé en ce que** le moyen de stockage de la substance active se présente sous forme d'un cylindre (15) dont l'axe central est évidé pour former un réservoir cylindrique (16) destiné à recevoir la substance active, et dont l'une des extrémités est destinée à être connectée directement à l'extrémité proximale (3) du tube, tandis que l'autre extrémité est obturée par un bouchon (24) étanche mobile axialement dans le réservoir cylindrique (16) sous l'effet de la tige (19) d'un piston (18).

14. Appareil selon la revendication 13, **caractérisé en ce que** le réservoir cylindrique reçoit une ampoule de substance active à paroi déformable, l'extrémité de l'ampoule étant connectée à l'extrémité proximale du tube.

## Patentansprüche

1. Vorrichtung, welche geeignet ist, die Abgabe eines Wirkstoffs direkt innerhalb der Gesamtheit oder eines Teils des menschlichen oder tierischen Zellgewebes sicherzustellen, und welche die Form eines hohlen Tubus (1) aufweist, der mit Löchern (5) versehen ist, die über die gesamte Länge der Wände ausgebildet sind, welche dazu bestimmt sind, mit dem Gewebe in Kontakt zu sein, und dessen distales Ende (2) verschlossen ist, während das proximale Ende (3) derart ausgebildet ist, dass es lösbare Verschlussmittel aufnimmt, **dadurch gekennzeichnet, dass** der Tubus geeignet ist, einem Druck von wenigstens 50 bar standzuhalten.

2. Vorrichtung nach Anspruch 1, **dadurch gekennzeichnet, dass** der Außendurchmesser des Tubus (1) zwischen 100 und 250 Mikrometern liegt, während der Innendurchmesser des Tubus (1) zwischen 50 und 150 Mikrometern liegt.

3. Vorrichtung nach einem der vorhergehenden Ansprüche, **dadurch gekennzeichnet, dass** der Außendurchmesser des Tubus gleich 200 Mikrometer ist, während der Innendurchmesser des Tubus gleich 100 Mikrometer ist.

4. Vorrichtung nach einem der Ansprüche 2 oder 3, **dadurch gekennzeichnet, dass** die Löcher (5) im Wesentlichen kreisförmig sind und einen Durchmesser aufweisen, der zwischen 30 und 70 Mikrometern, vorteilhafterweise bei 50 Mikrometern liegt.

5. Vorrichtung nach einem der Ansprüche 2 bis 4, **dadurch gekennzeichnet, dass** das den Tubus bildende Material aus der Gruppe umfassend rostfreien Stahl und Titan ausgewählt ist.

6. Vorrichtung nach Anspruch 1, **dadurch gekennzeichnet, dass** der Außendurchmesser des hohlen Tubus zwischen 300 und 700 Mikrometern liegt, während der Innendurchmesser zwischen 100 und 300 Mikrometern liegt.

7. Vorrichtung nach Anspruch 6, **dadurch gekennzeichnet, dass** die an den Wänden ausgebildeten Löcher im Wesentlichen kreisförmig sind und einen Durchmesser aufweisen, der zwischen 100 und 200 Mikrometern, vorteilhafterweise bei 150 Mikrometern liegt.

8. Vorrichtung nach einem der Ansprüche 6 oder 7, **dadurch gekennzeichnet, dass** der Tubus aus Polytetrafluorethylen (PTFE) gefertigt ist.

9. Vorrichtung nach einem der vorhergehenden Ansprüche, **dadurch gekennzeichnet, dass** die Löcher (5) spiralförmig mit einer Steigung von 0,5 bis 2 Zentimetern um den Teil des Tubus herum ausgebildet sind, welcher dazu bestimmt ist, mit dem zu behandelnden Organ (7) in Kontakt zu sein.

10. Vorrichtung nach einem der vorhergehenden Ansprüche, **dadurch gekennzeichnet, dass** die lösbaren Verschlussmittel in Form eines Pfropfens vorliegen, welcher dazu bestimmt ist, an das proximale Ende des Tubus geschraubt zu werden.

11. Osteosynthese-Element, das mit der Vorrichtung ausgestattet ist, die Gegenstand eines der Ansprüche 1 bis 10 ist.

12. Gerät für das Injizieren eines Wirkstoffs, das der Vorrichtung zugeordnet ist, welche Gegenstand von einem der Ansprüche 1 bis 10 ist, wobei das Gerät folgendes aufweist:
• einerseits ein Mittel zur Speicherung des Wirkstoffs, das geeignet ist, an das proximale Ende des Tubus angeschlossen zu werden;
• andererseits Mittel, die geeignet sind, das Injizieren des Wirkstoffs durch den Tubus (1) zu ermöglichen.

13. Gerät nach Anspruch 12, **dadurch gekennzeichnet, dass** das Mittel zur Speicherung des Wirkstoffs in Form eines Zylinders (15) vorliegt, dessen Mittelachse ausgehöhlt ist, um einen zylinderförmigen Behälter (16) zu bilden, welcher dazu bestimmt ist, den Wirkstoff aufzunehmen, und dessen eines Ende dazu bestimmt ist, direkt an das proximale Ende (3) des Tubus angeschlossen zu werden, während das andere Ende durch einen unter der Wirkung der Stange (19) eines Kolbens (18) in dem zylinderförmigen Behälter (16) axial beweglichen dichten Pfropfen (24) verschlossen ist:

14. Gerät nach Anspruch 13, **dadurch gekennzeichnet, dass** der zylinderförmige Behälter eine Wirkstoffampulle mit verformbarer Wand aufnimmt, wobei das Ende der Ampulle an das proximale Ende des Tubus angeschlossen wird.

## Claims

1. A device for delivering an active substance directly within all or some of a human or animal cell tissue, having the form of a hollow tube (1), equipped with perforations (5) formed over the entire length of the walls intended to be in contact with the tissue, and of which the distal end (2) is plugged, while the proximal end (3) is shaped in such a way as to accomodate removable closure means, **characterized in that** said tube is capable of withstanding a pressure of at least 50 bar.

2. The device as claimed in claim 1, **characterized in that** the outside diameter of the tube (1) is between 100 and 250 micrometers, while the inside diameter of the tube (1) is between 50 and 150 micrometers.

3. The device as claimed in one of the preceding claims, **characterized in that** the outside diameter of the tube is equal to 200 micrometers while the inside diameter of the tube is equal to 100 micrometers.

4. The device as claimed in one of claims 2 and 3, **characterized in that** the perforations (5) are substantially circular and have a diameter of between 30 and 70 micrometers, advantageously 50 micrometers.

5. The device as claimed in one of claims 2 to 4, **characterized in that** the material from which the tube is made is chosen from the group comprising stainless steel and titanium.

6. The device as claimed in claim 1, **characterized in that** the outside diameter of the hollow tube is between 300 and 700 micrometers, while the inside diameter is between 100 and 300 micrometers.

7. The device as claimed in claim 6, **characterized in that** the perforations formed on the walls are substantially circular and have a diameter of between 100 and 200 micrometers, advantageously of 150 micrometers.

8. The device as claimed in one of claims 6 and 7, **characterized in that** the tube is made of polytetrafluoroethylene (PTFE).

9. The device as claimed in one of the preceding claims, **characterized in that** the perforations (5) are formed in a spiral around the part of the tube that is intended to be in contact with the organ (7) that is to be treated, with a pitch of between 0.5 and 2 centimeters.

10. The device as claimed in one of the preceding claims, **characterized in that** the removable closure means are in the form of a plug intended to be screwed onto the proximal end of the tube.

11. An osteosynthesis element equipped with the device that is the subject of one of claims 1 to 10.

12. An appliance intended for injecting active substance combined with the device that is the subject of one of claims 1 to 10, **characterized in that** it has:
• on the one hand, a means of storing the active principle which can be connected to the proximal end of the tube;
• and, on the other hand, means capable of allowing the active substance to be injected through the tube (1).

13. The appliance as claimed in claim 12, **characterized in that** the means of storing the active substance is in the form of a cylinder (15), the central axis of which is hollowed out to form a cylindrical reservoir (16) intended to contain the active substance, and one of the ends of which is intended to be connected directly to the proximal end (3) of the tube, while the other end is plugged by a sealed plug (24) that can move axially inside the cylindrical reservoir (16) under the effect of the rod (19) of a piston (18).

14. The appliance as claimed in claim 13, **characterized in that** the cylindrical reservoir can contain a deformable-walled ampoule of active substance, the end of the ampoule being connected to the proximal end of the tube.
